# EUROPÄISCHE PATENTSCHRIFT

(11) **EP 0 449 860 B1**
(45) Veröffentlichungstag und Bekanntmachung des Hinweises auf die Patenterteilung: **15.06.1994**
(21) Anmeldenummer: 90900066.3
(22) Anmeldetag: 15.12.1989
(51) Int. Cl.: H04R 25/00, A61F 11/00

(54) **TINNITUS-MASKIERUNGSGERÄT**
TINNITUS MASKER
APPAREIL SERVANT A COUVRIR LE BOURDONNEMENT D'OREILLES

(30) Priorität: 22.12.1988 DE 8815877 U
(43) Veröffentlichungstag der Anmeldung: 09.10.1991
(73) Patentinhaber: JUNKER, Franz, D-76275 Ettlingen (DE)
(72) Erfinder: JUNKER, Franz, D-76275 Ettlingen (DE)
(74) Vertreter: Pfeifer, Hans-Peter, Dr., Dr. H.-P. Pfeifer Dr. P. Jany, Patentanwälte
(86) Internationale Anmeldenummer: DE8900776
(87) Internationale Veröffentlichungsnummer: WO9007251

(56) Entgegenhaltungen:
- DE-A- 3 027 791
- GB-A- 2 134 689
- SE-B- 428 860
- US-A- 4 222 393

## Beschreibung

Die Erfindung betrifft ein Tinnitus-Maskierungsgerät mit einer in einem Gehäuse angeordneten elektronischen Schaltung und einem Hörer zur Erzeugung eines Schallspektrums, das den Tinnitus des Patienten überdecken kann, wobei zur Einstellung der Schallintensität ein Lautstärkeregler vorgesehen ist.

Als Tinnitus wird eine Krankheit bezeichnet, bei der der Patient im Ohr oder Kopf Geräusche wahrnimmt, für die keine äußeren Ursachen vorhanden sind. Dies kann außerordentlich unangenehm sein und in schweren Fällen zu schweren geistigen und körperlichen Störungen führen.

In der wissenschaftlichen Literatur wird bereits seit vielen Jahren die Möglichkeit untersucht, das Tinnitus-Leiden dadurch zu lindern, daß man das Tinnitus-Geräusch mit einem dem Ohr zugeführten Schallsignal übertönt. Zahlreiche Untersuchungen wurden dabei von H. Feldmann angestellt, wobei der Einfluß von Breitbandgeräuschen, Schmalbandgeräuschen und reinen Tönen untersucht wurde. Hingewiesen sei insbesondere auf folgende Arbeiten:
H. Feldmann: "Homolaterale und kontralaterale Verdekkung von subjektiven Ohrgeräuschen durch Breitbandgeräusche, Schmalbandgeräusche und reine Töne"; Arch. klin. exp. Ohr.-Nas.-Kehlk.-Heilk. 194, 460-465 (1969).
H. Feldmann: "Homolateral and contralateral masking of tinnitus by noise-bands and pure tones"; Audiology, 10, 138-144 (1971).
H. Feldmann: "Homolateral and contralateral masking of tinnitus, Proceedings of the 1st International Tinnitus Seminar"; J. Laryngol. Otol., Suppl. 4, S. 60-70 (1981).

Um dem Patienten unabhängig von einem großen und aufwendigen Gerät auch unterwegs ein Mittel zur Linderung seines Leidens zur Verfügung zu stellen, werden in neuerer Zeit miniaturisierte Tinnitus-Maskierungsgeräte ("Tinnitus-Masker") angeboten. Sie sind entweder eigenständige Geräte oder in Hörgeräte eingebaut. Auch ein Signalgeber, der auf ein konventionelles Hörgerät zusätzlich aufsteckbar ist, wurde bereits vorgeschlagen (DE-A-3027791).

Bei solchen Geräten wird Wert darauf gelegt, daß das Maskierungsgeräusch breitbandiger als das zu verdeckende Ohrgeräusch ist (R. Schönweiler: "Ohrgeräusche: Ursachen, Bewertung und Therapie"; Dtsch. med. Wschr. 111, 1489-1494, insbesondere 1492 linke Spalte, (1986). Die bekannten Tinnitus-Masker arbeiten deshalb mit einem Rauschsignal, also einem breiten, undifferentierten Frequenzspektrum, das sich praktisch über das ganze hörbare Schallspektrum erstreckt und üblicherweise ein breites Maximum zwischen etwa 1000 Hz und 5000 Hz hat. Die genaue Kontur des Rauschspektrums wird von dem jeweiligen Hersteller des Gerätes festgelegt. Der Patient kann meist nur die Lautstärke mit einem entsprechenden Regler verändern. Bei manchen Geräten ist auch eine Beeinflussung des Frequenzverlaufes mit Hilfe eines Tonreglers möglich. Die Wirkung dieses Tonreglers besteht darin, einen bestimmten Teil des Frequenzspektrums (insbesondere tiefe Töne) anzuheben oder abzusenken.

Aus der SE-B 428 860 und der GB-A-2 134 689 sind Tinnitus-Masker bekannt, bei denen das Frequenzspektrum des Maskierungsgeräusches in größerem Umfang verändert werden kann. Zu diesem Zweck ist bei der SE-B-428 860 eine von dem eigentlichen Signalgenerator trennbare Programmiereinheit vorgesehen, in der in digitaler Form Informationen über das gewünschte Geräuschspektrum eingegeben werden. Diese Informationen werden von Fall zu Fall auf einen in der Signalgeneratoreinheit integrierten Speicher übertragen. Dadurch können verhältnismäßig komplizierte Rauschsignal-Spektren in Abhängigkeit von dem Speicherinhalt erzeugt werden. Eine unmittelbare Anpassung des Geräuschspektrums am Gerät durch den Patienten selbst ist nicht möglich. Außerdem ist das Gerät elektronisch aufwendig. Auch bei dem in der GB-A-2 134 689 beschriebenen System ist eine digitale Programmierung des Masker-Geräusches vorgesehen. Hierzu ist ein separates Charakterisierungssystem erforderlich. Mit Hilfe dieses Charakterisierungssystems soll von einem Fachmann das für den betreffenden Patienten geeignete Maskierungs-Geräuschspektrum festgelegt werden. Der Masker wird entsprechend programmiert. Auch dieses digital funktionierende Verfahren ist aufwendig. Eine Einstellung durch den Patienten selbst ist nicht vorgesehen.

Der vorliegenden Erfindung liegt die Aufgabe zugrunde, einen Tinnitus-Masker mit verbesserter Wirksamkeit zur Verfügung zu stellen, wobei zugleich der elektronische Aufwand möglichst gering sein soll, um eine weitgehende Miniaturisierung zu ermöglichen.

Diese Aufgabe wird bei einem Tinnitus-Maskierungsgerät der eingangs bezeichneten Art dadurch gelöst, daß die elektronische Schaltung so ausgebildet ist, daß das vom Hörer erzeugte Schallspektrum ein Linienspektrum mit einem Grundton enthält, wobei die Frequenz des Grundtons vom Benutzer einstellbar ist.

Überraschenderweise hat sich gezeigt, daß mit einer einfachen harmonischen Schallschwingung, welche sowohl hinsichtlich der Schallintensität, als auch hinsichtlich der Frequenz des Grundtons vom Benutzer selbst eingestellt werden kann, eine sehr gute Wirkung bei vielen Tinnitus-Patienten erreicht werden kann. Die Patienten passen die Frequenzeinstellung und die Lautstärke jeweils ihren Bedürfnissen an, wobei sich zeigt, daß beim gleichen Patienten zu verschiedenen Zeitpunkten recht unterschiedliche Frequenzen zur optimalen Wirkung führen. Dabei erweisen sich gelegentlich auch Frequenzen als wirksam, die außerhalb des normalerweise hörbaren Spektrum liegen. Die Frequenz ist deswegen besonders bevorzugt zwischen 0,125 und 20 kHz einstellbar. Die Erfindung umfaßt jedoch auch einfachere Ausführungsformen, bei denen die Einstellmöglichkeit nur zwischen 3000 und 8000 Hz, vorzugsweise zwischen 1 kHz und 12 kHz liegt. In jedem Fall sollte sich die Untergrenze des Einstellbereiches von dessen Obergrenze um mindestens einen Faktor 2, bevorzugt um einen Faktor 4, unterscheiden.

Gute Ergebnisse lassen sich sowohl mit einer frequenzvariablen harmonischen Schwingung (Sinusschwingung), als auch mit einer frequenzvariablen nichtharmonischen periodischen Schwingung, insbesondere Rechteckschwingung, erzielen.

Gemäß einer besonders bevorzugten Ausführungsform ist ein für den Benutzer zugänglicher Schalter vorgesehen, durch den, vorzugsweise alternativ, verschiedene periodische Schwingungen (jeweils frequenzvariabel) vom Benutzer auf den Hörer geschaltet werden können.

Die Erfindung wird im folgenden anhand eines in den Figuren schematisch dargestellten Ausführungsbeispiels näher erläutert, es zeigen:
- Fig. 1: ein erfindungsgemäßes Tinnitus-Maskierungsgerät in perspektivischer Ansicht,
- Fig. 2: ein Blockschaltbild einer für ein erfindungsgemäßes Tinnitus-Maskierungsgerät geeigneten elektronischen Schaltung
- Fig. 3-Fig. 6: graphische Darstellungen von vier verschiedenen Spektren eines erfindungsgemäßen Tinnitus-Maskierungsgerätes.

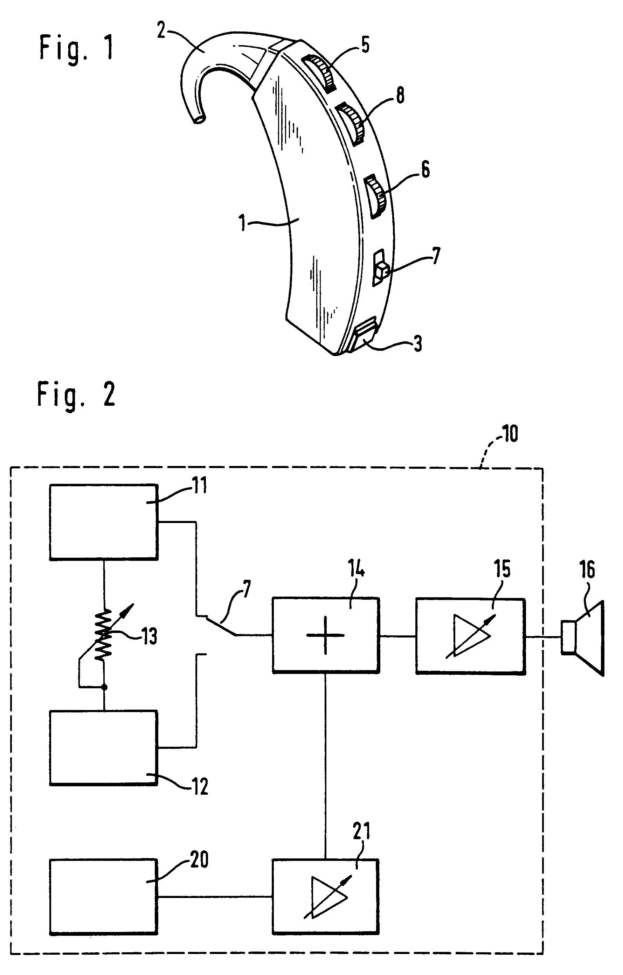

Das in Fig. 1 dargestellte Tinnitus-Maskierungsgerät hat ein Gehäuse 1 ähnlich einem hinter dem Ohr zu tragenden Hörgerät mit einem aufsteckbaren Hörkanal 2. Der Hörkanal 2 wird über eine in der Figur nicht dargestellte, in der Hörgeräteakustik gebräuchliche Gehörgangsplastik mit dem Ohr des Patienten verbunden. An dem Gehäuse 1 ist wie üblich ein Batteriefach 3 vorgesehen.

Ein Einstellknopf 5 dient zur Einstellung der Schallintensität. Ein weiterer Einstellknopf 6 ist zur Einstellung der Grundtron-Frequenz vorgesehen. Ein Umschalter 7 dient zur Umschaltung zwischen zwei verschiedenen periodischen Schwingungen, insbesondere zwischen Sinus und Rechteck. Ein Einstellknopf 8 ist zur Einstellung der Intensität eines überlagerten Rauschsignals vorgesehen.

Die in Fig. 2 dargestellte Schaltung 10 weist einen Sinusgenerator 11, einen Rechteckgenerator 12 und ein als Frequenzeinsteller dienendes Potentiometer 13 auf, welches von dem Einstellknopf 6 betätigt wird. Über den Schalter 7 und einen Addierer 14 läßt sich wahlweise das Sinussignal des Generators 11 oder das Rechtecksignal des Generators 12 an einen Verstärker mit variabler Verstärkung 15 anlegen. Der Verstärkungsfaktor ist mit Hilfe des Knopfes 5 einstellbar. Das Ausgangssignal des Verstärkers 15 liegt an dem Hörer 16 an, von dem ein Schallsignal erzeugt und in den Hörkanal 2 geleitet wird.

Gemäß einer bevorzugten Ausführungsform ist zusätzlich ein Rauschgenerator 20 vorgesehen, der über einen variablen Verstärker 21 an dem Addierer 14 anliegt und mit dem periodischen Signal aus dem Sinusgenerator 11 oder dem Rechteckgenerator 12 überlagert wird. Der Verstärkungsfaktor des Verstärkers 21 (also allgemein gesprochen die Intensität des überlagerten Rauschsignals) ist vorzugsweise vom Benutzer mit Hilfe des Einstellknopfes 8 einstellbar. Dabei sollte die Intensität des Rauschsignals stets geringer als die Intensität des periodischen Signals sein.

Einzelheiten zum Aufbau der in Fig. 2 dargestellten Bestandteile der Schaltung werden hier nicht beschrieben, weil dem elektronisch vorgebildeten Fachmann zahlreiche verschiedene Realisierungsmöglichkeiten für Signalgeneratoren, Addierer und Verstärker aus der einschlägigen Fachliteratur ohne weiteres zur Verfügung stehen. Es sind auch fertige integrierte Schaltungen, die diese Bestandteile enthalten, kommerziell erhältlich. Die Schaltung hat einen sehr einfachen Aufbau hat. Dadurch ist eine Miniaturisierung in einem sehr kleinen Gehäuse möglich.

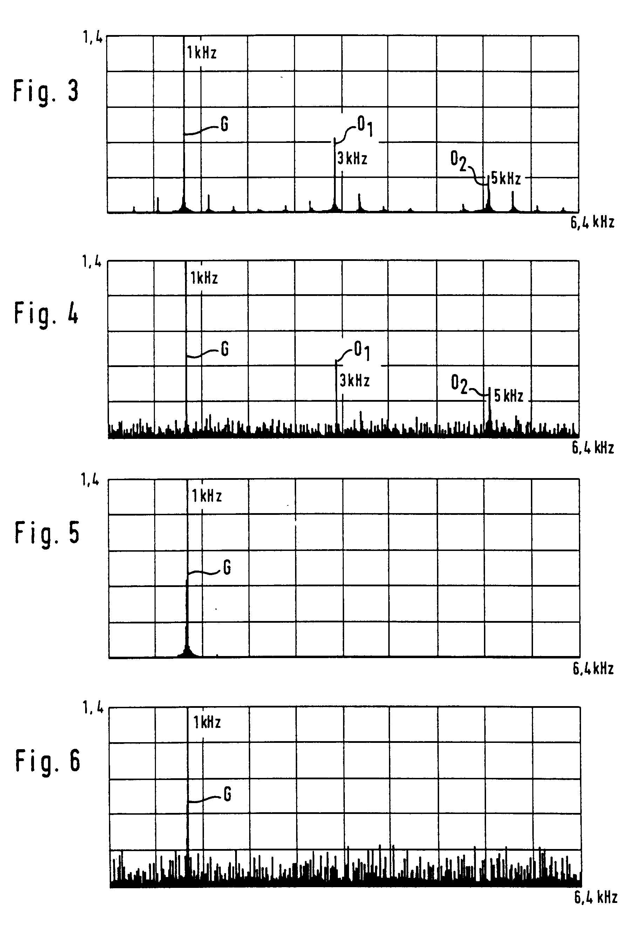

Die Figuren 3 bis 6 zeigen vier verschiedene Schallsignal-Spektren eines erfindungsgemäßen Tinnitus-Maskierungsgerätes, wobei jeweils die Frequenz auf der Abszisse und die Amplitude auf der Ordinate aufgetragen ist. Die Frequenzen der Spektrallinien und die Frequenz am rechten Rand des Spektrums sind jeweils angegeben.

Fig. 3 zeigt das Linienspektrum eines 1 kHz-Rechtecksignals. Die höchste Amplitude hat die Grundton-Linie G bei 1 kHz. Oberton-Linien O₁ und O₂ sind bei 3 kHz und 5 kHz zu erkennen. Weitere Frequenzlinien mit einer geringeren Amplitude sind darauf zurückzuführen, daß die verwendete Schaltung kein ideales Rechteck erzeugt. Dies ist im Rahmen der vorliegenden Erfindung unschädlich, solange das Schallspektrum nur ein deutlich erkennbares Linienspektrum enthält und die Grundtonfrequenz wie oben beschrieben in einem weiten Frequenzbereich vom Benutzer einstellbar ist.

Wie oben erwähnt, kann es zweckmäßig sein, dem Linienspektrum ein Rauschspektrum verhältnismäßig geringer Amplitude zuzumischen. Ein solcher Fall ist in Fig. 4 dargestellt, wobei das in Fig. 3 abgebildete 1 kHz-Rechteckspektrum mit einem Rauschen überlagert ist, dessen Amplitude erheblich geringer als die der Frequenzlinie des zweiten Obertons O₂. Allgemein sollte das Rauschspektrum jedenfalls eine erheblich geringere Amplitude als die Grundton-Linie (vorzugsweise weniger als 50 %, besonders bevorzugt weniger als 20%) haben.

Fig. 5 und Fig. 6 zeigen zu den Figuren 3 und 4 analoge Darstellungen für den Fall, daß das Linienspektrum auf einer harmonischen Schwingung (Sinussignal) basiert. In Fig. 5 wird das Spektrum von der Linie der 1 kHz-Grundschwingung G beherrscht. Das in Fig. 6 zusätzlich überlagerte Rauschsignal hat eine Amplitude, die im Mittel weniger als 20 % der Amplitude des Grundtons beträgt.

## Patentansprüche

1. Tinnitus-Maskierungsgerät mit
einer in einem Gehäuse (1) angeordneten elektronischen Schaltung und einem Hörer (16) zur Erzeugung eines Schallspektrums, das den Tinnitus des Patienten überdecken kann,
wobei zur Einstellung der Schallintensität ein Lautstärkeregler (5) vorgesehen ist,
**dadurch gekennzeichnet**, daß
die elektronische Schaltung (10) so ausgebildet ist, daß das von dem Hörer (16) erzeugte Schallsprektrum ein Linienspektrum mit einem Grundton enthält, wobei die Frequenz des Grundtons an dem Gerät von dem Patient selbst einstellbar ist.

2. Tinnitus-Maskierungsgerät nach Anspruch 1, **dadurch gekennzeichnet**, daß die Frequenz des Grundtons zwischen 0,125 und 20 kHz einstellbar ist.

3. Tinnitus-Maskierungsgerät nach Anspruch 1 oder 2, **dadurch gekennzeichnet**, daß die elektronische Schaltung (10) einen Schaltungsteil (12) zur Erzeugung einer Sinusschwingung enthält, die an den Hörer (2) anschaltbar ist.

4. Tinnitus-Maskierungsgerät nach einem der vorhergehenden Ansprüche, **dadurch gekennzeichnet**, daß die elektronische Schaltung (10) einen Schaltungsteil (12) zur Erzeugung einer nichtharmonischen periodischen Schwingung enthält, die an den Hörer anschaltbar ist.

5. Tinnitus-Maskierungsgerät nach Anspruch 4, **dadurch gekennzeichnet**, daß ein für den Benutzer zugänglicher Schalter (7) vorgesehen ist, durch den verschiedene periodische Schwingungen an den Hörer (16) angelegt werden.

6. Tinnitus-Maskierungsgerät nach einem der Ansprüche 4 oder 5, **dadurch gekennzeichnet**, daß die nichtharmonische periodische Schwingung eine Rechteckschwingung ist.

7. Tinnitus-Maskierungsgerät nach einem der vorhergehenden Ansprüche, **dadurch gekennzeichnet**, daß die elektronische Schaltung einen Schaltungsteil (20) zur Erzeugung eines Rauschens enthält, welches dem Linienspektrum überlagert wird.

8. Tinnitus-Maskierungsgerät nach einem der vorhergehenden Ansprüche, **dadurch gekennzeichnet**, daß die Rauschintensität vom Benutzer einstellbar ist.

9. Tinnitus-Maskierungsgerät nach einem der vorhergehenden Ansprüche, **dadurch gekennzeichnet**, daß sich die Untergrenze des Einstellbereiches der Frequenz des Grundtons von der Obergrenze des Einstellbereiches der Frequenz des Grundtons um mindestens einen Faktor 2, bevorzugt mindestens einen Faktor 4, unterscheidet.

## Claims

1. A tinnitus masking device comprising an electronic circuit, which is arranged in a housing (1), and a receiver (16) for the generation of a sound spectrum which can mask the patient's tinnitus, wherein a volume control means (5) is provided for the adjustment of the sound intensity, characterised in that the electronic circuit (10) is designed in such manner that the sound spectrum generated by the receiver (16) contains a line spectrum with a basic tone, where the frequency of the basic tone is adjustable on the device by the patient himself.

2. A tinnitus masking device as claimed in Claim 1, characterised in that the frequency of the basic tone is adjustable between 0.125 and 20 kHz.

3. A tinnitus masking device as claimed in Claim 1 or 2, characterised in that the electronic circuit (10) comprises a circuit component (12) for the generation of a sine-wave oscillation which can be switched to the receiver (2).

4. A tinnitus masking device as claimed in one of the preceding claims, characterised in that the electronic circuit (10) comprises a circuit component (12) for the generation of a non-harmonic, periodic oscillation which can be switched to the receiver.

5. A tinnitus masking device as claimed in Claim 4, characterised in that a switch (7) accessible to the user is provided, by which different periodic oscillations are fed to the receiver (16).

6. A tinnitus masking device as claimed in one of Claims 4 or 5, characterised in that the non-harmonic, periodic oscillation is a rectangular-wave oscillation.

7. A tinnitus masking device as claimed in one of the preceding claims, characterised in that the electronic circuit comprises a circuit component (20) which generates a noise which is superimposed upon the line spectrum.

8. A tinnitus masking device as claimed in one of the preceding claims, characterised in that the noise intensity is adjustable by the user.

9. A tinnitus masking device as claimed in one of the preceding claims, characterised in that the lower limit of the adjustment range of the frequency of the basic tone differs from the upper limit of the adjustment range of the frequency of the basic tone by at least a factor of 2, and preferably at least a factor of 4.

## Revendications

1. Appareil servant à couvrir le bourdonnement d'oreilles, comprenant un circuit électronique logé dans un boîtier (1) et un écouteur (16) pour la génération d'un spectre sonore susceptible de couvrir le bourdonnement d'oreilles du patient, un régulateur de volume sonore (5) étant prévu pour le réglage de l'intensité acoustique, **caractérisé en ce** que le circuit électronique (10) est conçu de telle façon que le spectre acoustique généré par l'écouteur (16) contient un spectre de raies avec un son fondamental, la fréquence du son fondamental pouvant être réglée par l'utilisateur.

2. Appareil servant à couvrir le bourdonnement d'oreilles selon la revendication 1, caractérisé en ce que la fréquence du son fondamental peut être réglée entre 0,125 et 20 kHz.

3. Appareil servant à couvrir le bourdonnement d'oreilles selon l'une des revendications 1 ou 2, caractérisé en ce que le circuit électronique (10) comprend un élément de circuit (12) pour la génération d'une oscillation sinusoïdale qui peut être appliquée à l'écouteur (2).

4. Appareil servant à couvrir le bourdonnement d'oreilles selon l'une des revendications précédentes, caractérisé en ce que le circuit électronique (10) comprend un élément de circuit (12) pour la génération d'une oscillation périodique non harmonique qui peut être appliquée à l'écouteur.

5. Appareil servant à couvrir le bourdonnement d'oreilles selon la revendication 4, caractérisé en ce qu'il comprend un commutateur (7) accessible à l'utilisateur et permettant d'appliquer à l'écouteur (16) différentes oscillations périodiques.

6. Appareil servant à couvrir le bourdonnement d'oreilles selon l'une des revendications 4 ou 5, caractérisé en ce que l'oscillation périodique non harmonique est une oscillation rectangulaire.

7. Appareil servant à couvrir le bourdonnement d'oreilles selon l'une des revendications précédentes, caractérisé en ce que le circuit électronique comprend un élément de circuit (20) pour la génération d'un bruit lequel est superposé au spectre de raies.

8. Appareil servant à couvrir le bourdonnement d'oreilles selon l'une des revendications précédentes, caractérisé en ce que l'intensité du bruit peut être réglée par l'utilisateur.

9. Appareil servant à couvrir le bourdonnement d'oreilles selon l'une des revendications précédentes, caractérisé en ce que la limite inférieure de la plage de réglage de la fréquence du son fondamental diffère d'au moins un facteur 2, de préférence d'au moins un facteur 4, de la limite supérieure de la plage de réglage de la fréquence du son fondamental.
